# EUROPEAN PATENT SPECIFICATION

(11) **EP 0 690 044 B1**
(45) Date of publication and mention of the grant of the patent: **03.05.2000**
(21) Application number: 95109997.7
(22) Date of filing: 27.06.1995
(51) Int. Cl.: C07C 231/12, C07C 233/18, C11D 1/83, C07C 231/02, C11D 1/06

(54) **Process for producing amidoether carboxylic acid or salt thereof, and surface active agent mixture containing the same**
Verfahren zur Herstellung von Amidoethercarbonsäure oder deren Salz und diese enthaltende oberflächenaktive Mischung
Procédé pour la préparation d'acide amidoéthercarboxylique ou de son sel et mélange d'agent de surface le contenant

(30) Priority: 27.06.1994 JP 14439594; 02.08.1994 JP 18133394
(43) Date of publication of application: 03.01.1996
(73) Proprietor: KAO CORPORATION, Chuo-ku, Tokyo (JP)
(72) Inventor: Terasaki, Hiroyuki, c/o Kao Corp., Res. Lab., Wakayama-shi, Wakayama-ken (JP); Fujiu, Akira, c/o Kao Corp., Res. Lab., Wakayama-shi, Wakayama-ken (JP); Isobe, Kazuo, c/o Kao Corp., Res. Lab., Wakayama-shi, Wakayama-ken (JP); Azuma, Toshikazu, c/o Kao Corp., Res. Lab., Wakayama-shi, Wakayama-ken (JP); Nishikawa, Hideyo, c/o Kao Corp., Res. Lab., Wakayama-shi, Wakayama-ken (JP); Imamura, Takashi, c/o Kao Corp., Res. Lab., Wakayama-shi, Wakayama-ken (JP)
(74) Representative: Hansen, Bernd, Dr. Dipl.-Chem.

(56) References cited:
- EP-A- 0 102 118
- EP-A- 0 215 504
- EP-A- 0 219 893
- EP-A- 0 236 984
- EP-A- 0 386 826
- EP-A- 0 560 570
- PATENT ABSTRACTS OF JAPAN vol. 010, no. 136 (C-347), 20 May 1986 & JP-A-60 261536 (SANYO KASEI KOGYO KK), 24 December 1985,
- PATENT ABSTRACTS OF JAPAN vol. 95, no. 008, 29 September 1995 & JP-A-07 138592 (KAO CORP), 30 May 1995,
- PATENT ABSTRACTS OF JAPAN vol. 018, no. 268 (C-1202), 23 May 1994 & JP-A-06 041580 (NIPPON OIL & FATS CO LTD), 15 February 1994,

## Description

This invention relates to a process for industrially producing a surface active agent mixture containing an amidoether carboxylic acid or a salt thereof, more particularly a surface active agent mixture which hardly irritates the skin, and exhibits excellent foaming properties.

### Description of the Related Art

Anionic surface active agents, such as alkylsulfates, polyoxyethylene alkylsulfates, alkylbenzenesulfonates, and α-olefin sulfonates, have widely been used in detergents because of their high foaming properties. However, these anionic surface active agents are more or less irritant to the skin and cause skin roughening when used continuously.

On the other hand, alkyl saccharide type surface active agents, sulfosuccinic acid type surface active agents, ether carboxylic acid surface active agents, amidoether carboxylic acid type surface active agents, and the like are known to be low-irritant surface active agents. However, alkyl saccharide type surface active agents, though highly foaming, feels rough at washing or rinsing. To reduce the rough feeling, a combined use with a conditioner such as a cationic polymer has been proposed as disclosed in Japanese Patent Application Laid-Open 2-42013, but it is not technically easy to incorporate a large quantity of such a conditioner into a shampoo, etc. Since sulfosuccinic acid type surface active agents alone have poor foaming properties, a combined use with other surface active agents has been suggested as disclosed in Japanese Patent Application Laid-Open 2-218797. In practice, they are often used in combination with other surface active agents. Commercially available ether carboxylic acid type or amidoether carboxylic acid type surface active agents also have poor foaming properties so that their utility, in those detergents of which high foaming properties are demanded, has been confined to use as auxiliary surface active agents.

With respect to the amidoether carboxylic acid surface active agents above referred to, EP-A-0102118 discloses toiletries containing an amidoether carboxylic acid represented by formula:

R-CO-NH-(C₂H₄O)ₖ-CH₂COOH

wherein R-CO- represents a residue of at least one aliphatic carboxylic acid having 6 to 22 carbon atoms; and k represents a number averaging 2 to 10,
or a salt thereof which is mild to the skin and yet lathers well.

The above-mentioned amidoether carboxylic acid or a salt thereof can be prepared, for example, by the process disclosed in Japanese Patent Application Laid-Open 63-291996 (corresponding to EP-A-0219893), which consists of aminolysis of oil or fat with ethanolamine in the presence of an alkali metal or an alkaline earth metal and ethoxylation followed by carboxymethylation of the resulting mixture of a fatty acid ethanolamide and glycerin. According to this process, however, since the aminolysis product contains glycerin, the final product inevitably contains glycerin, an ethylene oxide adduct of glycerin and its carboxymethylated compound in addition to the aminoether carboxylic acid. As a result, the final product has an insufficient content of the amidoether carboxylic acid salt, the effective surface active component, and therefore does not always lather satisfactorily. While glycerin and its derivatives may be removed by converting the amidoether carboxylic acid salt to an acid form, followed by washing with water, this method results in a serious reduction in yield and is not deemed industrially advantageous.

A process starting with a fatty acid is also known, in which a fatty acid is reacted with ethanolamine to obtain a fatty acid ethanolamide, which is subjected to ethoxylation and then carboxymethylation. However, since the amidation reaction between a fatty acid and ethanolamine generally requires a high temperature, usually 120° C or higher, the resulting ethanolamide is apt to be colored. It follows that the amidoether carboxylic acid obtained by the subsequent ethoxylation and carboxymethylation assumes such a further deteriorated hue that would make the product difficult to be used practically in detergents, etc. Another problem associated with this process is that the amidation reaction aiming at production of a fatty acid ethanolamide is accompanied with esterification of the hydroxyl group of the once formed fatty acid ethanolamide with the fatty acid to form an amidoester compound (R'CON(R'')CH₂CH₂OCOR'), resulting in reduction in yield of the amidoether carboxylic acid.

The aforementioned amidoether carboxylic acid type surface active agents are commercially sold by Chem Y, Germany under a series of trade names "Akypo". The commercial products contain not only an amidoether carboxylic acid and the starting amidoether but also impurities originating in the starting material, i.e., polyoxyethylene glyceryl ether, polyoxyethylene glyceryl ether carboxylic acid, inorganic salts, etc.

Known techniques relating to application of amidoether carboxylic acid type surface active agents to detergents include a detergent comprising a combination of an amidoether carboxylic acid, a polyoxyethylene alkylsulfate, a fatty acid diethanolamide, a higher alcohol alkylene oxide adduct and an acyl sarcosinate. (European Patent 102118); a method of combining an amidoether carboxylic acid surface active agent and a polyoxyethylene alkylsulfate (European Patent 215504); foaming bath agents, shampoo agents, shower agents, and the like comprising a combination of an amidoether carboxylic acid prepared from fat and oil, a laurylsulfate, a lauryl ether sulfate, an alkyl ether carboxylic acid, and a quaternized product obtained by reacting lauric acid and glycidyltrimethylammonium chloride (Japanese Patent Publication 63-291996 corresponding to EP-A-0219893); and a detergent mainly comprising soap and having incorporated thereinto an amidoether carboxylic acid or an alkyl ether carboxylic acid salt (U.S. Patent 4,865,757). However, any of the conventional detergents was still unsatisfactory in foaming properties.

It is obvious that an alkaline detergent tends to roughen the skin, and it is desirable that a detergent should be neutral to acidic in order to allay irritation to the skin.

Accordingly, it has been demanded to develop a neutral to acidic detergent composition which is less irritant to the skin and excellent in foaming properties.

### Summary of the Invention

An object of the present invention is to provide a process for producing a surface active agent mixture containing an amidoether carboxylic acid or a salt thereof.

A further object of the present invention is to provide a surface active agent mixture, which hardly irritates the skin and has high foaming properties.

As a result of extensive investigations, the present inventors have found that a high foaming surface active agent mixture containing an amidoether carboxylic acid or a salt thereof can be produced by using a fatty acid lower alkyl ester as a starting material. It was found that amidation of the fatty acid lower alkyl ester can be carried out at a low temperature so that no coloration occurs in the subsequent ethoxylation and carboxymethylation.

The present invention has been completed based on this finding.

The process of the present invention is represented by the following reaction scheme: 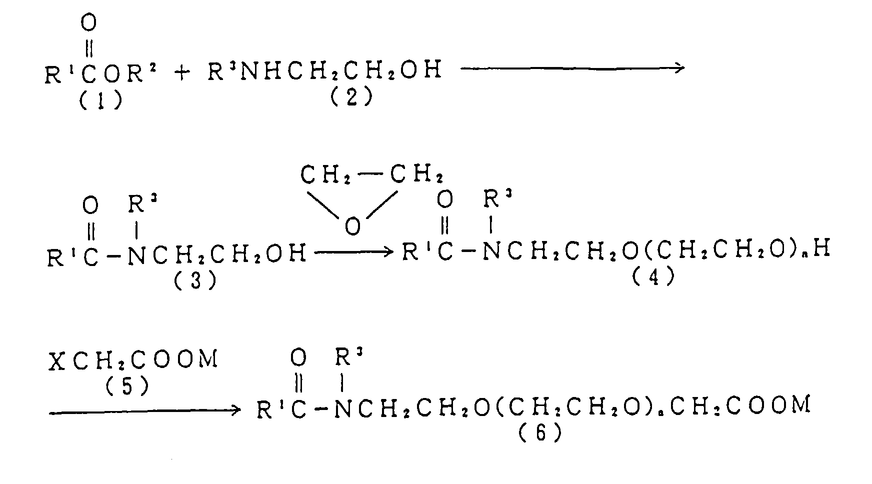 wherein R¹ represents a straight-chain or branched alkyl or alkenyl group having 5 to 23 carbon atoms or a phenyl group substituted with the alkyl group; R² represents a straight- chain or branched alkyl group having 1 to 4 carbon atoms; R³ represents a hydrogen atom, or a straight-chain or branched alkyl group having 1 to 5 carbon atoms, wherein M represents a hydrogen atom, an alkali metal, an alkaline earth metal, an ammonium group, an alkanolamine or a basic amino acid; n represents a number of from 0.5 to 20 on an average; and X represents a halogen atom.

The present invention relates to a process for producing a surface active agent mixture containing an amidoether carboxylic acid or a salt thereof represented by formula (VI) comprising reacting a fatty acid alkyl ester represented by formula (I) with ethanolamine or a derivative thereof represented by formula (II) (hereinafter inclusively referred to as ethanolamine (II)) to form a fatty acid ethanolamide represented by formula (III), reacting the fatty acid ethanolamide with a desired amount of ethylene oxide to obtain an amidoether represented by formula (IV), and reacting the amidoether with a monohalogenoacetic acid or a salt thereof represented by formula (V) in the presence of a solid alkali.

### Detailed Description of the Invention

The process according to the present invention will be described below.

In formula (I) representing the fatty acid lower alkyl ester that can be used as a starting material in the process, R¹ represents a straight-chain or branched alkyl or alkenyl group having 5 to 23 carbon atoms or a phenyl group substituted with the alkyl group, preferably a straight-chain or branched alkyl or alkenyl group having 7 to 17 carbon atoms or a phenyl group substituted with the alkyl group, still preferably a straight-chain or branched alkyl group having 7 to 17 carbon atoms. Examples of preferred groups of R¹ are a heptyl group, a nonyl group, an undecyl group, a tridecyl group, a pentadecyl group, a heptadecyl group, and a heptadecenyl group, with an undecyl group and a tridecyl group being most preferred.

R² includes a methyl group, an ethyl group, an n-propyl group, an isopropyl group, an n-butyl group, and an isobutyl group, with methyl and ethyl groups being preferred. A methyl group is most preferred.

Particularly preferred examples of the fatty acid lower alkyl esters (I) are methyl octanoate, methyl decanoate, methyl laurate, methyl myristate, methyl palmitate, methyl stearate, methyl isostearate, methyl oleate, coconut oil, palm oil, and fatty acid methyl esters of beef tallow origin. These fatty acid lower alkyl esters (I) are available at low prices on an industrial scale, and the commercially available products, even those obtained from fats and oils, have high purity with little glycerin.

In formula (II) representing ethanolamine or a derivative thereof, R³ represents a hydrogen atom or a straight-chain or branched alkyl group having 1 to 5 carbon atoms, such as a methyl group, an ethyl group, an isopropyl group, an n-butyl group, an isobutyl group, or an n-pentyl group. Particularly preferred as R³ is a hydrogen atom or an alkyl group having 1 to 3 carbon atoms.

Specific examples of the ethanolamine (II) are ethanolamine, N-methylethanolamine, and N-ethylethanolamine, with ethanolamine being preferred.

The reaction between the fatty acid alkyl ester (I) and the ethanolamine (II) can be carried out at a temperature of 60 to 120° C, preferably 70 to 110° C, under a pressure of 1 to 760 mmHg, preferably 30 to 100 mmHg, for a period of 2 to 24 hours in the absence or presence of an alkali catalyst, such as sodium methylate. The reaction mixture undergoes substantially no coloration as far as the reaction is performed under the above conditions. The reaction ratio of the ethanolamine (II) to the fatty acid alkyl ester (I), (II)/(I), preferably ranges from 1.0 to 1.1, still preferably from 1.0 to 1.05. An equimolar ratio or a slight excess of the ethanolamine (II) is most preferred.

Ethylene oxide is then added to the resulting fatty acid ethanolamide (III) at a temperature of 150° C or lower to obtain the amidoether (IV). The addition reaction may be carried out in the absence of additional catalyst or in the presence of an alkali catalyst, such as sodium hydroxide or potassium hydroxide, which is added to the fatty acid ethanolamide (III) in an amount of from 0.05 to 10 mol% based on the fatty acid ethanolamide (III). Addition of ethylene oxide is preferably conducted at 150° C or lower; for a reaction temperature exceeding 150° C causes decomposition of the amide moiety and formation of an ester component, leading to coloration. The number of moles of ethylene oxide to be added (n) is in the range of from 0.5 to 20 on an average, preferably from 0.5 to 10, still preferably from 0.5 to 5.

In formula (V) representing a monohalogenoacetic acid or a salt thereof (hereinafter simply referred to as the monohalogenoacetic acid or a salt thereof (V)) which is used in the next reaction, M includes sodium, potassium, magnesium, calcium, monoethanolamine, diethanolamine, triethanolamine, arginine or lysine. Specific examples of the monohalogenoacetic acid or a salt thereof (V) include monohalogenoacetic acid alkali metal salts, such as sodium monochloroacetate, sodium monobromoacetate, potassium monochloroacetate, and potassium monobromoacetate, with sodium monochloroacetate being preferred. The terminology "solid alkali" as used herein means an alkali to which substantially no water has been added. The solid alkali may have such a water content as in commercially available products. Examples of suitable solid alkalis include sodium alcoholates, alkali hydroxides, and alkali carbonates, such as sodium methylate, sodium ethylate, sodium hydroxide, potassium hydroxide, sodium carbonate, and potassium carbonate, with sodium hydroxide being most preferred.

The reaction between the amidoether (IV) and the monohalogenoacetic acid or a salt thereof (V) can be carried out by adding 0.1 to 3.0 mol, preferably 0.1 to 1.5 mol, of the monohalogenoacetic acid or a salt thereof (V) per mole of the amidoether (IV) in the presence of the solid alkali at 50 to 90° C, preferably 60 to 80° C, over a period of 1 to 24 hours, preferably 3 to 6 hours. When the monohalogenoacetic acid salt is used, the solid alkali is needed in an amount of 0.8 to 1.5 mol, preferably 1.0 to 1.2 mol per mol of the monohalogenoacetic acid. When the monohalogenoacetic acid salt is used, about 1 mol of the solid alkali is further needed to neutralize the monohalogenoacetic acid. If desired, produced water may be removed during the reaction by, for example, reducing the reaction pressure or passing nitrogen through the reaction system. From the standpoint of yield, the mixture of the monohalogenoacetic acid or a salt thereof (V) and the solid alkali are preferably added in divided portions rather than all at once.

If desired, after completion of the reaction between the amidoether (IV) and the momohalogenoacetic acid or a salt thereof (V), the reaction mixture may be aged for several hours under the same conditions as used for the addition or some different conditions to complete the reaction.

The thus obtained crude reaction mixture can be worked up by various methods, for example, as follows.
(1) Being strongly alkaline, the crude reaction mixture is diluted with water to a solids content of 30% on dry basis and adjusted to pH 7 preferably with hydrochloric acid so as to improve storage stability.
(2) When purification is needed, a water-hydrochloric acid mixture is heated to about 90° C, and the crude reaction mixture is added thereto followed by stirring. The solution is allowed to stand, and the oily layer is separated and washed. The oily layer recovered contains the desired product in the form of a free acid.
(3) When a product of a salt form is desired, an aqueous alkali solution is added to the purified product obtained by method (2) at about 50° C to convert the product into its salt.
(4) In order to improve the hue or odor of the product, the reaction mixture is treated with a reducing agent, such as sodium borohydride, either during or after completion of carboxymethylation.

The amidoether carboxylic acid or a salt thereof represented by formula (VI) (hereinafter simply referred to as the amidoether carboxylic acid or a salt thereof (VI)) is more satisfactory in terms of foaming properties and foam stability when it contains the amidoether (IV) than when it is 100% pure. The mixing ratio of the amidoether carboxylic acid or a salt thereof (VI) and the amidoether (IV), (VI)/(IV), is in the range of from 99.9/0.1 to 1/9, particularly from 9/1 to 3/7, by weight. The (VI)/(IV) mixing ratio can be so controlled by adjusting the amount of the monohalogenoacetic acid or a salt thereof (V) to be used in the above reaction.

The reaction product obtained by the process of the present invention has a reduced content of impurities, such as glycerin derivatives, as have been by-produced in Japanese Patent Application Laid-Open 63-291996 (corresponding to EP-A-219893). On the other hand, the amidoether carboxylic acid or a salt thereof containing 0.1 to 5% by weight, for example, 0.5 to 4% by weight, of a fatty acid or its neutral salt as byproducts exhibits low irritation and high foaming properties and is useful as a neutral to acidic surface active agent mixture. If desired, a fatty acid or a salt thereof can be added to the reaction product obtained by the process of the present invention to provide a composition.

The surface active agent mixture obtainable in the present invention will then be illustrated below.

The surface active agent mixture is obtainable by the above-mentioned process and comprises an amidoether carboxylic acid or a salt thereof represented by formula (VI): 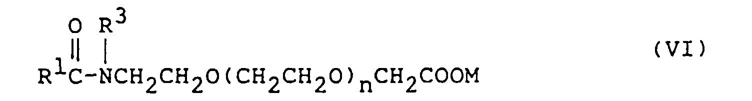 wherein R¹, R³, M and n are as defined above;
and an amidoether represented by formula (IV): 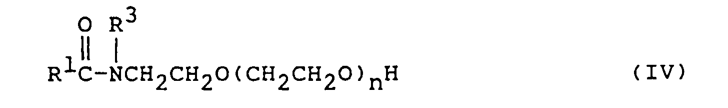 wherein R¹, R³, and n are as defined above,
wherein
the total amount of the amidoether carboxylic acid or a salt thereof (VI) and the amidoether (IV) is 50% by weight or more based on the solid content of the surface active agent mixture; and
the ratio of the amidoether carboxylic acid or a salt thereof (VI) to the amidoether (IV) is from 99:1 to 10:90 by weight.

Preferred examples of the amidoether carboxylic acid or a salt thereof (VI) in the surface active agent mixture include various compounds represented by formula (VI). Specifically, preferred of them are lauric monoethanolamide polyoxyethylene ether acetic acid (n=1 to 10), myristic monoethanolamide polyoxyethylene ether acetic acid (n=1 to 10), palmitic monoethanolamide polyoxyethylene ether acetic acid (n=1 to 10), stearic monoethanolamide polyoxyethylene ether acetic acid (n=1 to 10), and salts thereof. Still preferred of them are lauric monoethanolamide polyoxyethylene ether acetic acid (n=2 to 7) and myristic monoethanolamide polyoxyethylene ether acetic acid (n=2 to 7).

Preferred examples of the amidoether (IV) in the surface active agent mixture include various compounds represented by formula (IV). Specifically, preferred of them are lauric monoethanolamide polyoxyethylene ether (n=1 to 10), myristic monoethanolamide polyoxyethylene ether (n=1 to 10), palmitic monoethanolamide polyoxyethylene ether (n=1 to 10), and stearic monoethanolamide polyoxyethylene ether (n=1 to 10). Still preferred of them are lauric monoethanolamide polyoxyethylene ether (n=2 to 7) and myristic monoethanolamide polyoxyethylene ether (n=2 to 7).

The surface active agent mixture obtainable in the present invention preferably contains the amidoether carboxylic acid or a salt thereof (VI) and the amidoether (IV) in a total amount of at least 50%, preferably 60% or more, still preferably 70% or more, most preferably 80% or more, by weight based on the solids content of the surface active agent mixture.

The weight ratio of the amidoether carboxylic acid or a salt thereof (VI) to the amidoether (IV), (VI):(IV), preferably ranges from 99:1 to 10:90, more preferably from 95:5 to 60:40, still preferably from 92:8 to 70:30.

The surface active agent mixture obtainable in the present invention comprises the amidoether carboxylic acid or a salt thereof (VI) and the amidoether (IV) as main components. The surface active agent mixture obtainable in the present invention may contain glycerin or a derivative thereof represented by the following formula (VII) (hereinafter inclusively referred to as glycerin derivative (VII)), preferably in an amount of less than 5% by weight based on the solids content of the surface active agent mixture. 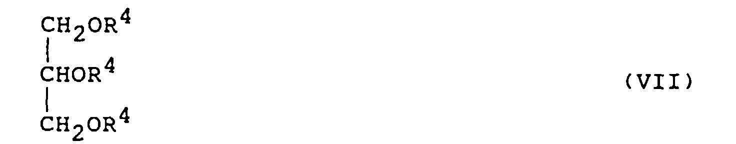 wherein R⁴ independently represents a hydrogen atom, -(CH₂CH₂O)₁CH₂COOM or -(CH₂CH₂O)ₘH, wherein l and m independently represent a number of from 1 to 20, and M is as defined above.

The content of glycerin derivative (VII) in the surface active agent mixture is preferably less than 5%, still preferably less than 3%, by weight based on the solids content of the mixture. It is the most preferred that the surface active agent mixture contains substantially no glycerin derivative (VII). If the content of glycerin derivative (VII) exceeds 5% by weight based on the solids content, the foaming properties of the mixture are considerably reduced. With regards to inorganic salts, e.g., sodium chloride, which are impurities other than glycerin derivative (VII), the smaller the content, the better.

The surface active agent mixture obtainable in the present invention can be prepared by the above-described process according to the present invention; for the resulting reaction mixture is less colored and contains substantially no glycerin derivatives as impurities. Where the other processes are followed, for example, fat and oil having a coconut oil fatty acid composition as a starting material is directly converted into an alkanolamide, and the resulting alkanolamide is alkoxylated and then carboxylmethylated, glycerin derivatives originating in the fat and oil are produced in a considerable amount, resulting in low yields of the compounds (VI) and (IV), which is not practically preferable. Where a fatty acid as a starting material is reacted with an alkanolamine to form an alkanolamide, the reaction mixture unfavorably suffers from considerable coloration due to the high reaction temperature of the amidation. The ratio of the amidoether carboxylic acid (VI) and the amidoether (IV) in the surface active agent mixture obtainable in the present invention can be adjusted by properly selecting the reaction conditions, such as the molar ratio of the monohalogenoacetic acid or a salt thereof (V) (e.g., monohalogenoacetic acid alkali salt) to the amidoether (IV), the manner of mixing, and the like.

It is preferable that the surface active agent mixture obtainable in the present invention further contains a fatty acid or a salt thereof having a pH of neutral to acid.

The surface active agent mixture will be described in detail hereinafter.

In the surface active agent mixture containing the fatty acid or a salt thereof, the amidoether carboxylic acids or salts thereof and the amidoethers can be used either individually or in combination of two or more thereof.

The proportion of the amidoether carboxylic acid or a salt thereof and the amidoether in the surface active agent mixture containing the fatty acid or a salt thereof is not particularly limited but is preferably not less than 3% by weight, still preferably not less than 5% by weight, in view of foaming properties.

Next, the fatty acid or a salt thereof will be described. The fatty acid or a salt thereof includes straight-chain or branched and saturated or unsaturated fatty acids having 6 to 32 carbon atoms or salts thereof. Examples of component (B) include caproic acid, caprylic acid, capric acid, undecanoic acid, lauric acid, tridecanoic acid, myristic acid, pentadecanoic acid, palmitic acid, heptadecanoic acid, stearic acid, oleic acid, elaidic acid, linoleic acid, linolenic acid, arachic acid, behenic acid, lignoceric acid, and alkali metal salts, alkaline earth metal salts, ammonium salts, alkanolamine salts or basic amino acid salts of these acids. Preferred among them are lauric acid, myristic acid, palmitic acid, and alkali metal salts, ammonium salts, alknaolamine salts or basic amino acid salts of these acids.

These fatty acids or salts thereof may be used either individually or in combination of two or more thereof. The proportion of the fatty acid or a salt thereof in the surface active agent mixture is not particularly limited but preferably ranges from 0.1 to 45% by weight, particularly from 0.5 to 20% by weight.

The ratio of [the amidoether carboxylic acid or a salt thereof and the amidoether] to [the fatty acid or a salt thereof] in the surface active agent mixture is not particularly limited but preferably ranges from 99.9:0.1 to 25:75, still preferably from 95:5 to 60:40 by weight.

The surface active agent mixture containing the fatty acid or a salt thereof is preferably neutral to acidic, i.e. has a pH value of ≤ 7.4. Specifically, it preferably has a pH of 3.0 to 7.4, more preferably 4.0 to 7.0, most preferably 5.0 to 7.0, as measured as an aqueous solution containing 5% by weight of the active components. If the pH is less than 3.0, the composition has reduced foaming properties and is irritant to the skin. If the pH exceeds 7.4, the foaming properties are reduced, and irritation to the skin becomes stronger as the pH further increases. Therefore, the above range is preferred. The pH adjustment can be effected by addition of a known acidic or alkaline chemical employed in conventional detergent compositions.

The surface active agent mixture may contain surface active agents other than the amidoether carboxylic acids, such as anionic surface active agents and nonionic surface active agents, in addition to the aforesaid components as far as the effects of the present invention are not impaired.

Examples of the anionic surface active agents include polyoxyalkylene alkyl ether acetates, alkylsulfates, and polyoxyalkylene alkyl ether sulfates. Examples of the nonionic surface active agents include polyglycerin fatty acid esters, polyoxyalkylene alkyl ethers, alkylamine oxides, and fatty acid polyhydric alcohol esters.

The surface active agent mixture may further contain components generally used in detergent compositions as far as the effects of the present invention are not impaired. Such components include water-soluble polymers such as polysaccharides, e.g., methyl cellulose, hydroxyethyl cellulose, carboxyvinyl polymer, and xantham gum; viscosity modifiers such as polyoxyalkylene sorbitan esters, polyoxyethylene glycol distearate, and ethanol; chelating agents such as ethylenediaminetetraacetic acid (EDTA) and phosphoric acid salts; antiseptics such as methyl p-hydroxybenzoate and butyl p-hydroxybenzoate; nutrient components such as vitamins or precursors thereof; animal or vegetable extracts such as lecithin and gelatin, or derivatives thereof; fine particles of polymers such as nylon and polyethylene; antiinflammatory agents such as potassium glycyrrhizinate; bactericidal agents or antidandruff agents such as Triclosan, Triclocarban, Octopirox, and Zinc Pyrithione; antioxidants such as dibutylhydroxytoluene; pearly luster imparting agents, ultraviolet absorbers, pH adjusting agents, dyes, perfumes, and the like.

The surface active agent mixture is applicable as not only a detergent for hair or the skin but a detergent for various purposes, such as detergents for dishes or clothing. It is preferable that the total amount of surface active agents inclusive of the amidoether doether carboxylic acid or a salt thereof and the in the surface active agent mixture is at least 30% by weight in the case of a solid preparation, at least 20% by weight in the case of a paste preparation, and at least 10% by weight in the case of a liquid preparation.

As has been fully described, the present invention makes it possible to easily prepare an amidoether carboxylic acid or a salt thereof with a satisfactory hue and high purity. Accordingly, the amidoether carboxylic acid or a salt thereof obtained by the present invention is useful as a main active agent or a lather booster of detergents which are keenly required to cause no irritation to the skin and to have a satisfactory hue, foaming properties, and foam stability, such as shampoos, body shampoos, bubble bath agents, soap, dish-washing detergents, and toothpastes.

The present invention also provides a surface active agent mixture which causes little irritation to the skin, exhibits excellent foaming properties, and produces creamy foam.

The present invention will now be illustrated in greater detail with reference to Examples, but it should be understood that the present invention is not deemed to be limited thereto. Unless otherwise indicated, all the percents are given by weight.

### EXAMPLE 1

A mixture of 214 g (1 mol) of methyl laurate, 62 g (1.02 mol, 1.02 equivalents to methyl laurate) of monoethanolamine, and a 30% methanol solution of sodium methylate (0.1% based on methyl laurate) was heated at 90° C and 66 mbar (50 mmHg) for 5 hours. To the resulting product was added 2 mol of ethylene oxide at 100 to 110° C and a gauge pressure of 0 to 3.5 atm over a period of 40 minutes.

The resulting reaction mixture weighing 331.4 g was heated to 70 to 75° C, and 174.8 g (1.5 mol) of sodium monochloroacetate (hereinafter abbreviated as SMCA) and 65.2 g (1.63 mol) of solid NaOH were each added thereto in 5 equally divided portions according to the schedule shown below.

| Time | SMCA | NaOH |
|---|---|---|
| (hr) | (%) | (%) |
| 0 (at the start of the reaction) | 20 | 20 |
| 1 | 20 | 20 |
| 2 | 20 | 20 |
| 3 | 20 | 20 |
| 4 | 20 | 20 |

After the final addition, the reaction mixture was aged for 1 hour. Then, the reaction temperature was raised to 85° C, 5.3 g of water was added thereto to crush unreacted sodium monochloroacetate, followed by further aging for 1 hour to give 592.1 g of the product. To the reaction mixture was added 500 g of water, and the mixture was adjusted to pH 7 with a 36% aqueous HCl solution at 50° C. Since the mixture would gel when left as such, it was diluted with water until it turned to a clear solution.

### EXAMPLE 2

The reaction of Example 1 was repeated, except for using a mixture of 93.2 g (0.8 mol) of SMCA and 34.8 g (0.87 mol) of solid NaOH.

After addition of SMCA and NaOH, the reaction mixture was aged for 1 hour. Thereafter, the reaction temperature was raised to 85° C, 5.3 g of water was added thereto, and the mixture was further aged for 1 hour to obtain 456.4 g of the product. To the reaction mixture was added 500 g of water, and the mixture was adjusted to pH 2.8 with a 36% aqueous HCl solution at 90° C, followed by stirring for 1 hour. The reaction mixture was allowed to stand for 1 hour for phase separation to obtain 409.8 g of a compound of an acid form. The resulting acid form compound was adjusted to pH 7 with a 30% aqueous NaOH solution and diluted with water until it became clear.

### COMPARATIVE EXAMPLE 1

Purified coconut oil weighing 510.6 g (2.23 mol) was melted at 35° C. A mixture of the coconut oil, 138.8 g (2.28 mol, 1.02 equivalent to coconut oil) of monoethanolamine, and 5.1 g of a 30% methanol solution of sodium methylate (NaOMe) was heated at 70° C for 2 hours and then at 105° C for 6 hours. To the resulting product was introduced 298 g (6.77 mol) of ethylene oxide at 100 to 110° C under a gauge pressure of 0-3,5 bar (0 to 3.5 atm) over a period of 30 minutes.

The resulting reaction mixture (674.7 g) was heated to 70 to 75°C, and 281.7 g (2.4 mol) of SMCA and 105.1 g (2.6 mol) of solid NaOH were each added thereto in 5 equally divided portions according to the schedule shown below.

| Time | SMCA | NaOH |
|---|---|---|
| (hr) | (%) | (%) |
| 0 | 20 | 20 |
| (at the start of the reaction) | | |
| 1 | 20 | 20 |
| 2 | 20 | 20 |
| 3 | 20 | 20 |
| 4 | 20 | 20 |

After the final addition, the reaction mixture was aged for 1 hour. Then, the reaction temperature was raised to 85° C, and 5 g of water was added thereto, followed by further aging for 1 hour to give 1038.7 g of the product.

To the reaction mixture was added 500 g of water, and the mixture was adjusted to pH 7 with a 36% aqueous HCl solution at 50° C and diluted with water until it turned to a clear solution.

### COMPARATIVE EXAMPLE 2

A mixture of 200 g (1 mol) of lauric acid, 62 g (1.02 mol, 1.02 equivalent to lauric acid) of monoethanolamine, and a 30% methanol solution of NaOMe (0.1% based on lauric acid) was dehydrated by heating at 150° C for 6 hours. To the resulting product was introduced 2 mol of ethylene oxide at 100 to 110° C under a gauge pressure of 0-3,5 bar (0 to 3.5 atm) over a period of 40 minutes.

The resulting reaction mixture (331.4 g) was heated to 70 to 75° C, and 174.8 g (1.5 mol) of SMCA and 65.2 g (1.63 mol) of solid NaOH were added thereto in divided portions in the same manner as in the foregoing Examples and Comparative Example.

After addition of SMCA and NaOH, the reaction mixture was aged for 1 hour. Thereafter, the reaction temperature was raised to 85° C, 5.3 g of water was added thereto, and the mixture was further aged for 1 hour to obtain 595.3 g of the product.

To the reaction mixture was added 500 g of water, and the mixture was adjusted to pH 7 with a 36% aqueous HCl solution at 50° C and diluted with water until it became a clear solution.

In Table 1 below are shown the composition of the product obtained in Examples and Comparative Examples (analyzed by alkali Epton method, ion chromatography, gas chromatography, fat and oil analysis, and the like), foam volume with a solids content of 1% on dry basis, and the hue on dilution to a solids content of 30% on dry basis.

The foam volume was evaluated as follows. In a measuring cylinder having an inner diameter of 65 mm and a height of 270 mm was put 100 ml of a detergent solution prepared by using 4° DH hard water, 0.5% of lanolin was added thereto, and the thus prepared test solution was adjusted at 30° C. A reversible rotation stirrer was set over the liquid level in such a manner that the stirring blades rotates in the solution. The test solution was agitated by reversible stirring for 5 minutes (max: 1000 rpm). After 30 seconds from the cessation of stirring, the volume of the foam was measured.

**TABLE 1**

| | | Examples | | Comparative Examples | |
|---|---|---|---|---|---|
| | | 1 | 2 | 1 | 2 |
| Composition of Product (wt%) | Amidoether Carboxylic Acid (AEC) | 23.1 | 18.1 | 13.8 | 14.7 |
| | Sodium Laurate * | 3.0 | 0.5 | 2.9 | 5.0 |
| | NaCl | 7.5 | 1.1 | 8.2 | 6.4 |
| | SMCA | 0 | 0 | 0 | 0.4 |
| | Sodium Glycolate | 1.0 | 0 | 0.5 | 1.2 |
| | Sodium Diglycolate | 5.6 | 0 | 1.2 | 4.1 |
| | Amidoether (AE) | 2.7 | 17.8 | 14.9 | 3.0 |
| | Other Components | 0 | 0 | 0 | 2.0 |
| | Glycerin and its derivatives | 0 | 0 | 17.5 | 0 |
| | Water | 57.0 | 62.5 | 41.0 | 63.2 |
| (AEC+AE)/Dry solids × 100 | | 60.5 | 95.7 | 48.6 | 48.1 |
| Foam volume [1% (on dry solid basis) solution] (mℓ) | | 250 | 270 | 10 | 190 |
| Hue [30% (on dry solid basis) solution] (APHA) | | 100 | 90 | 100 | G7 |

| | | | | | |
|---|---|---|---|---|---|
| Note: *In Compavative Example 1, fatty acid sodium salts of coconut composition was used. | | | | | |

It can be seen from Table 1 that the amidoether carboxylic acid salts obtained by the process of the present invention have a satisfactory hue and excellent foaming properties.

### EXAMPLE 3

A mixture of 214.4 g (1 mol) of methyl laurate, 61.7 g (1.02 mol) of monoethanolamine, and 15.3 g of a 30% methanol solution of sodium methoxide was heated at 90° C and 66 mbar (50 mmHg) for 5 hours. To the resulting product was introduced 88.2 g (2 mol) of ethylene oxide at 100 to 110° C and a gauge pressure of 0-3,5 bar (0 to 3.5 atm).

The resulting reaction mixture weighing 331 g was heated to 70 to 75° C, and 174.8 g (1.5 mol) of SMCA and 65.2 g of solid sodium hydroxide were added thereto over 4 hours. SMCA and sodium hydroxide were each divided into 5 equal portions, and each portions were added at the start of the reaction and after 1, 2, 3 and 4 hours from the start of the reaction. After the final addition, the reaction mixture was aged for 1 hour. Then, the reaction temperature was raised to 85° C, and 5.3 g of water was added thereto, followed by further aging for 1 hour to give 592 g of the carboxymethylized reaction mixture. To the reaction mixture was added 500 g of water, and the mixture was adjusted to pH 2.8 with a 36% aqueous solution of hydrochloric acid at 90° C, followed by stirring for 1 hour. The reaction mixture was allowed to stand for 1 hour for phase separation to obtain 545 g of a compound of an acid form. The resulting acid form compound was adjusted to pH 7 with a 30% aqueous NaOH solution, and water was added thereto till the solution became clear thereby to obtain amidoether derivative mixture 1 shown in Table 2 below.

### EXAMPLES 4 TO 8

Amidoether derivative mixtures 2, 3 and 4 shown in Table 2 were obtained in the same manner as in Example 3. SMCA was added in amounts of 1.5 mol, 1.5 mol and 1.4 mol, respectively, per mol of the amidoether. The solid sodium hydroxide was added in amounts of 1.63 mol, 1.63 mol and 1.52 mol, respectively, per mol of the amidoether. Further, amidoether derivative mixture 5 shown in Table 2 was prepared in the same manner as in Example 3 except that the conversion to an acid form and purification were not conducted and the reaction mixture was merely adjusted to pH 7 with a 30% aqueous NaOH solution. The acid form product obtained in Example 3 was neutralized to pH of 6 to 7 with a 30% aqueous magnesium hydroxide emulsion, and water was added thereto till the solution became clear thereby to obtain the amidoether derivative mixture 6 shown in Table 2.

### COMPARATIVE EXAMPLE 3

After a carboxymethylized reaction mixture was obtained in the same manner as in Example 3, the reaction mixture was added with ethanol so as to precipitate a solid. Thereafter, it was filtered, and the filter cake was washed with ethanol to remove amidoether. Further, after the product was made into an acid form as in Example 3, it was neutralized with a 30% sodium hydroxide solution thereby to obtain the amidoether derivative mixture 7.

### COMPARATIVE EXAMPLE 4

An amidoether was obtained in the same manner as in Example 3, and the amidoether was reacted with SMCA and solid sodium hydroxide under the condition that 0.05 mol of SMCA and 0.05 mol of solid sodium hydroxide were added per mol of the amidoether. Thereafer the reaction mixture was treated in the same manner in Example 3 thereby to obtain the amidoether derivative mixture 8.

**TABLE 2**

| Example No. | | | Compound (VI) | Compound (IV) | Content on Solid Basis (wt%) | | | | | (VI):(IV) |
|---|---|---|---|---|---|---|---|---|---|---|
| | | | | | (VI) | (IV) | (VI)+(IV) | (VII) | Balance* | |
| Examples | 3 | Amidoether Derivative Mixture 1 | R¹:C₁₁H₂₃ | R¹:C₁₁H₂₃ | 82 | 14 | 96 | 0 | 4 | 85 : 15 |
| | | | R³:H | R³:H | | | | | | |
| | | | M :Na | | | | | | | |
| | | | n :2 | n :2 | | | | | | |
| | 4 | Amidoether Derivative Mixture 2 | R¹:C₁₁H₂₃ | R¹:C₁₁H₂₃ | 77 | 18 | 95 | 0 | 5 | 81 : 19 |
| | | | R³:H | R³:H | | | | | | |
| | | | M :Na | | | | | | | |
| | | | n :4 | n :4 | | | | | | |
| | 5 | Amidoether Derivative Mixture 3 | R¹:C₁₃H₂₇ | R¹:C₁₃H₂₇ | 70 | 23 | 93 | 0 | 7 | 75 : 25 |
| | | | R³:H | R³:H | | | | | | |
| | | | M :Na | | | | | | | |
| | | | n :5 | n :5 | | | | | | |
| | 6 | Amidoether Derivative Mixture 4 | R¹:C₁₇H₃₅ | R¹:C₁₇H₃₅ | 60 | 35 | 95 | 0 | 5 | 63 : 37 |
| | | | R³:H | R³:H | | | | | | |
| | | | M :Na | | | | | | | |
| | | | n :9 | n :9 | | | | | | |
| | 7 | Amidoether Derivative Mixture 5 | R¹:C₁₁H₂₃ | R¹:C₁₁H₂₃ | 49 | 12 | 61 | 0 | 39 | 80 : 20 |
| | | | R³:H | R³:H | | | | | | |
| | | | M :Na | | | | | | | |
| | | | n :2 | n :2 | | | | | | |
| | 8 | Amidoether Derivative Mixture 6 | R¹:C₁₁H₂₃ | R¹:C₁₁H₂₃ | 82 | 14 | 96 | 0 | 4 | 85:15 |
| | | | R³:H | R³:H | | | | | | |
| | | | M :Mg | | | | | | | |
| | | | n :2 | n :2 | | | | | | |
| Comparative Examples | 3 | Amidoether Derivative Mixture 7 | R¹:C₁₁H₂₃ | R¹:C₁₁H₂₃ | 96 | 1< | 96 | 0 | 4 | 100: 0 |
| | | | R³:H | R³:H | | | | | | |
| | | | M :Na | | | | | | | |
| | | | n :2 | n :2 | | | | | | |
| | 4 | Amidoether Derivative Mixture 8 | R¹:C₁₁H₂₃ | R¹:C₁₁H₂₃ | 4 | 90 | 94 | 0 | 6 | 4:96 |
| | | | R³:H | R³:H | | | | | | |
| | | | M :Na | | | | | | | |
| | | | n :4 | n :4 | | | | | | |

| | | | | | | | | | | |
|---|---|---|---|---|---|---|---|---|---|---|
| *: Sodium chloride, glycolic acid salts, etc. | | | | | | | | | | |

### EXAMPLES 9 TO 11 AND COMPARATIVE EXAMPLES 5 TO 8

Detergent compositions having the composition shown in Table 3 below were prepared and evaluated in terms of foam volume, foam quality, and skin irritation. The results obtained are shown in Table 3. The weight percents of the formulations given in Table 3 are for the effective content of the active agent (solids content as to amidoether carboxylic acid salts). The pH adjustment was conducted using an aqueous sodium hydroxide solution and citric acid.

### Method of Evaluation:

One gram of a test detergent composition was applied to hairs of a healthy Japanese female (length: 15 cm) and made to lather for 1 minute. The foam volume and foam quality were scored by 20 specialized panel members according to standards A and judged from the average of 20 scores according to standards B. The results obtained are shown in Table 3.

Separately, a test detergent composition was applied to the healthy skin of 5 guinea pigs 4 times. The response after the 4th application was scored according to standards A, and the skin irritation was judged from the average of 5 scores according to standards B.
(1) Foam volume
   A. Standards of evaluation
      Very good lathering 4
      Good lathering 3
      Slightly poor lathering 2
      Poor lathering 1
   B. Standards of judgement
      Average score of 3.5 to 4.0 excellent
      Average score of 2.5 to 3.4 good
      Average score of 1.5 to 2.4 fair
      Average score of 1.0 to 1.4 poor
(2) Foam quality
   A. Standards of evaluation
      Creamy and very slippery 4
      Creamy and slippery 3
      Slightly rough and non-slippery 2
      Rough and non-slippery 1
   B. Standards of judgement
      Average score of 3.5 to 4.0 excellent
      Average score of 2.5 to 3.4 good
      Average score of 1.5 to 2.4 fair
      Average score of 1.0 to 1.4 poor
(3) Skin irritation
   A. Standards of evaluation
      Non-irritating
         (no response observed) 5
      Faintly irritating
         (slight erythema observed) 4
      Weakly irritating
         (obvious erythema observed) 3
      Medium-irritating
         (obvious erythema accompanied by edema) 2
      Strongly irritating
         (obvious erythema accompanied by necrosis or syncope) 1
   B. Standards of judgement
      Average score of 3.5 to 5.0 good
      Average score of 2.5 to 3.4 fair
      Average score of 1.0 to 2.4 poor

**TABLE 3**

| (wt%) | | | | | | | | |
|---|---|---|---|---|---|---|---|---|
| | | Examples | | | Comparative Examples | | | |
| | | 9 | 10 | 11 | 5 | 6 | 7 | 8 |
| Amidoether Derivative Mixture 1 | | 10 | 10 | - | - | - | - | - |
| Amidoether Derivative Mixture 6 | | - | - | 10 | - | - | - | - |
| Amidoether Derivative Mixture 7 | | - | - | - | 10 | - | - | - |
| Amidoether Derivative Mixture 8 | | - | - | - | - | 10 | - | - |
| Amidoether derivative mixture of Comparative Example 1 | | - | - | - | - | - | 10 | 20 |
| Lauric Acid | | 3 | - | - | 1 | - | 5 | 5 |
| Myristic Acid | | - | - | - | - | 2 | - | - |
| Coconut fatty Acid | | - | 2 | 3 | - | - | - | - |
| Ion-exchanged Water | | Balance | Balance | Balance | Balance | Balance | Balance | Balance |
| pH (5% Effective Content Aqueous Solution) | | 6.5 | 6.1 | 6.3 | 6.8 | 6.4 | 5.9 | 6.5 |
| Evaluation | Foam Volume | excellent | excellent | excellent | fair | poor | poor | poor |
| | Foam Quality | excellent | excellent | excellent | good | poor | poor | poor |
| | Skin Irritation | good | good | good | good | good | good | good |

### EXAMPLE 12

A shampoo having the following formulation and a pH of 6.5 was prepared. The shampoo was low-irritant to the skin and excellent in foaming properties and foam quality.

| Formulation: | |
|---|---|
| Amidoether derivative mixture 2 | 14% |
| Sodium laurate | 3% |
| Lauric diethanolamide | 3% |
| Citric acid | adequate amount |
| Sodium hydroxide | adequate amount |
| Perfume | 0.2% |
| Ion-exchanged water | balance |

### EXAMPLE 13

A body shampoo having the following formulation and a pH of 6.1 was prepared. The body shampoo was low-irritant to the skin and excellent in foaming properties and foam quality.

| Formulation: | |
|---|---|
| Amidoether derivative mixture 5 | 10% |
| Palmitic acid | 4% |
| Sodium polyoxyethylene (4.5) lauryl ether acetate (Akypo RLM45, produced by Chem Y) | 4% |
| Citric acid | adequate amount |
| Sodium hydroxide | adequate amount |
| Flavor | 0.2% |
| Ion-exchanged water | balance |

### EXAMPLE 14

A shampoo having the following formulation and a pH of 5.5 was prepared. The shampoo was low-irritant to the skin and excellent in foaming properties and touch of the hair after washing.

| Formulation: | |
|---|---|
| Amidoether derivative mixture 3 | 10% |
| Coconut fatty acid | 4% |
| Sodium polyoxyethylene (3) lauryl ether sulfate | 3% |
| Laurylamine oxide | 2% |
| Citric acid | adequate amount |
| Sodium hydroxide | adequate amount |
| Perfume | 0.2% |
| Ion-exchanged water | balance |

### EXAMPLE 15

A dish-washing detergent having the following formulation and a pH of 6.0 was prepared. The detergent was low-irritant to the skin and excellent in detergency, foaming properties, foam quality, and touch of the hands after dish washing.

| Formulation: | |
|---|---|
| Amidoether derivative mixture 1 | 15% |
| Lauric acid | 4% |
| Myristic acid | 2% |
| Citric acid | adequate amount |
| Sodium hydroxide | adequate amount |
| Perfume | 0.2% |
| Ion-exchanged water | balance |

### EXAMPLE 16

A body shampoo having the following formulation and a pH of 6.9 was prepared. The body shampoo was low-irritant to the skin and excellent in foaming properties and foam quality.

| Formulation: | |
|---|---|
| Amidoether derivative mixture 1 | 7% |
| Amidoether derivative mixture 6 | 6% |
| Lauric Acid | 3% |
| Hydroxyethyl cellulose | 0.7% |
| Perfume | 0.1% |
| Citric acid | adequate amount |
| Sodium hydroxide | adequate amount |
| Ion-exchanged water | balance |

### EXAMPLE 17

A dish-washing detergent having the following formulation and a pH of 7.0 was prepared. The detergent was low-irritant to the skin and excellent in touch of the hands after dish washing.

| Formulation: | |
|---|---|
| Amidoether derivative mixture 2 | 11% |
| Coconut fatty acid | 5% |
| Sodium polyoxyethylene (3) lauryl ether sulfate | 4% |
| Polyoxyethylene (10) lauryl ether | 2% |
| Ethanol | 2% |
| Magnesium chloride | 0.1% |
| Perfume | 0.2% |
| Citric acid | adequate amount |
| Sodium hydroxide | adequate amount |
| Ion-exchanged water | balance |

## Claims

1. A process for producing a surface active agent mixture comprising reacting a fatty acid alkyl ester represented by formula (I): 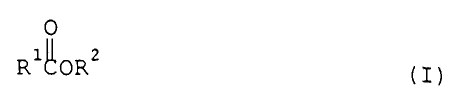 wherein R¹ represents a straight-chain or branched alkyl or alkenyl group having 5 to 23 carbon atoms or a phenyl group substituted with an alkyl group having 5 to 23 carbon atoms; and R² represents a straight-chain or branched alkyl group having 1 to 4 carbon atoms,
with ethanolamine or a derivative thereof represented by formula (II):
R³NHCH₂CH₂OH (II)
wherein R³ represents a hydrogen atom or a straight-chain or branched alkyl group having 1 to 5 carbon atoms,
to form a fatty acid ethanolamide represented by formula (III): 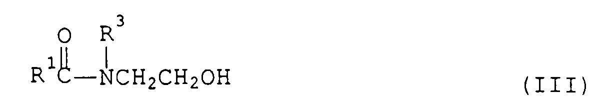 wherein R¹ and R³ are as defined above, reacting the fatty acid ethanolamide with ethylene oxide to obtain an amidoether represented by formula (IV): 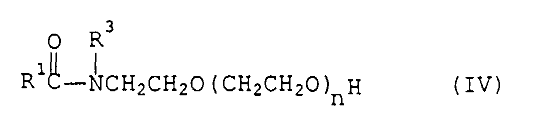 wherein R¹ and R³ are as defined above; and n represents a number of 0.5 to 20 on average,
and reacting the amidoether with a monohalogenoacetic acid or a salt thereof represented by formula (V):
XCH₂COOM (V)
wherein X represents a halogen atom; and M represents a hydrogen atom, an alkali metal, an alkaline earth metal, an ammonium group, an alkanolamine or a basic amino acid,
in the presence of a solid alkali, so as to produce the surface active agent mixture containing an amidoether carboxylic acid or a salt thereof represented by formula (VI): 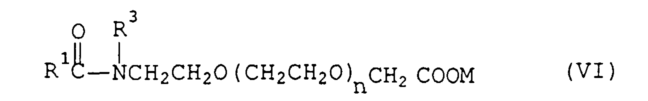 wherein R¹, R³, M and n are as defined above,
and the mixing ratio of the amidoether carboxylic acid or a salt thereof (VI) and the amidoether (IV), (VI)/(IV), being in the range of from 99.9/0.1 to 1/9 by weight.

2. The process according to claim 1, wherein R¹ is a straight-chain or branched alkyl group or an alkenyl group having 7 to 17 carbon atoms .

3. The process according to claim 1, wherein R³ is a hydrogen atom.

4. The process according to claim 1, wherein n is a number of from 0.5 to 10 on an average.

5. The process according to claim 1, wherein R¹ is a straight-chain alkyl group having 7 to 17 carbon atoms; R² is a methyl group; R³ is a hydrogen atom; and n is a number of from 0.5 to 5 on an average.

6. The process as claimed in claim 1, wherein the reaction of the fatty acid alkyl ester represented by formula (I) and the ethanolamine or the derivative thereof represented by formula (II) is carried out at a temperature of 60 to 120 °C under a pressure of 1,33 - 1033 mbar (1 to 760 mmHg).

7. The process according to claim 1, wherein the reaction of the fatty acid ethanolamide represented by formula (III) and ethylene oxide is carried out at a temperature of not higher than 150 °C.

8. The process according to claim 1, wherein the monohalogenoacetic acid or a salt thereof represented by formula (V) is used at a molar ratio of from 0.1 to 3.0 to the amidoether represented by formula (IV).

9. The process according to claim 8, wherein the reaction of the amidoether represented by formula (IV) and the monohalogenoacetic acid or a salt thereof represented by formula (V) is carried out at a temperature of 50 to 90 °C.

10. The process according to claim 1, wherein the monohalogenoacetic acid or salt represented by formula (V) and the solid alkali are added in divided portions and reacted with the amidoether represented by formula (IV).

## Patentansprüche

1. Verfahren zur Herstellung einer Tensidmischung, umfassend: Umsetzen eines Fettsäurealkylesters, dargestellt durch die Formel(I): 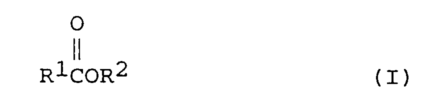 worin R¹ eine geradkettige oder verzweigte Alkyl- oder Alkenyl-Gruppe mit 5 bis 23 Kohlenstoffatomen oder eine Phenyl-Gruppe, die mit einer Alkyl-Gruppe mit 5 bis 23 Kohlenstoffatomen substituiert ist, darstellt; und R² eine geradkettige oder verzweigte Alkyl-Gruppe mit 1 bis 4 Kohlenstoffatomen darstellt,
mit Ethanolamin oder einem Derivat davon, dargestellt durch die Formel (II):
R³NHCH₂CH₂OH (II)
worin R³ ein Wasserstoffatom oder eine geradkettige oder verzweigte Alkyl-Gruppe mit 1 bis 5 Kohlenstoffatomen darstellt,
um ein Fettsäureethanolamid, dargestellt durch die Formel (III) zu bilden: 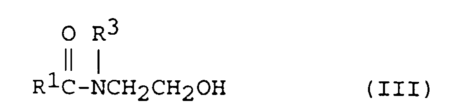 worin R¹ und R³ wie oben definiert sind,
Umsetzen des Fettsäureesterethanolamids mit Ethylenoxid, um einen Amidoether, dargestellt durch die Formel (IV) zu erhalten: 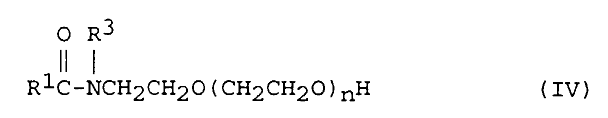 worin R¹ und R³ wie oben definiert sind; und n eine Zahl von durchschnittlich 0,5 bis 20 darstellt,
und Umsetzen des Amidoethers mit einer Monohalogenessigsäure oder einem Salz davon, dargestellt durch die Formel (V):
XCH₂COOM (V)
worin X ein Halogenatom darstellt; und M ein Wasserstoffatom, ein Alkalimetall, ein Erdalkalimetall, eine Ammonium-Gruppe, ein Alkanolamin oder eine basische Aminosäure darstellt,
in der Gegenwart einer festen Base, um eine Tensidmischung herzustellen, die eine Amidoethercarbonsäure oder ein Salz davon, dargestellt durch Formel (VI) enthält: 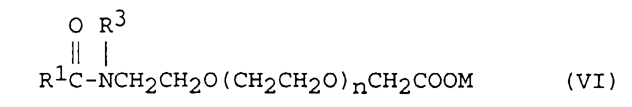 worin R¹, R³, M und n wie oben definiert sind,
und das Mischungsverhältnis der Amidoethercarbonsäure oder eines Salzes davon (VI) und des Amidoethers (IV), (VI)/(IV), im Bereich von 99,9/0,1 bis 1/9 Gewichtsteilen ist.

2. Verfahren gemäß Anspruch 1, worin R¹ eine geradkettige oder verzweigte Alkyl-Gruppe oder eine Alkenyl-Gruppe mit 7 bis 17 Kohlenstoffatomen ist.

3. Verfahren gemäß Anspruch 1, worin R³ ein Wasserstoffatom ist.

4. Verfahren gemäß Anspruch 1, worin n eine Zahl von durchschnittlich 0,5 bis 10 ist.

5. Verfahren gemäß Anspruch 1, worin R¹ eine geradkettige Alkyl-Gruppe mit 7 bis 17 Kohlenstoffatomen ist; R² eine Methyl-Gruppe ist; R³ ein Wasserstoffatom ist; und n eine Zahl von durchschnittlich 0,5 bis 5 ist.

6. Verfahren wie beansprucht in Anspruch 1, worin die Reaktion des Fettsäurealkylesters, dargestellt durch Formel (I), und des Ethanolamins oder des Derivats davon, dargestellt durch Formel (II), bei einer Temperatur von 60 bis 120°C unter einem Druck von 1,33 bis 1033 mbar (1 bis 760 mmHg) durchgeführt wird.

7. Verfahren gemäß Anspruch 1, worin die Reaktion des Fettsäureethanolamids, dargestellt durch Formel (III), und Ethylenoxid bei einer Temperatur von nicht mehr als 150°C durchgeführt wird.

8. Verfahren gemäß Anspruch 1, worin die Monohalogenessigsäure oder ein Salz davon, dargestellt durch Formel (V) in einem molaren Verhältnis von 0,1 bis 3,0 zu dem Amidoether, dargestellt durch Formel (IV), verwendet wird.

9. Verfahren gemäß Anspruch 8, worin die Reaktion des Amiodoethers, dargestellt durch Formel (IV), und der Monohalogenessigsäure oder eines Salzes davon, dargestellt durch Formel (V), bei einer Temperatur von 50 bis 90°C durchgeführt wird.

10. Verfahren gemäß Anspruch 1, worin die Monohalogenessigsäure oder Salz, dargestellt durch Formel (V) und die feste Base in getrennten Portionen zu dem Amidoether, dargestellt durch Formel (IV), gegeben und damit umgesetzt werden.

## Revendications

1. Procédé pour produire un mélange d'agents tensioactifs comprenant de faire réagir un ester alkylique d'acide gras représenté par la formule (I) : 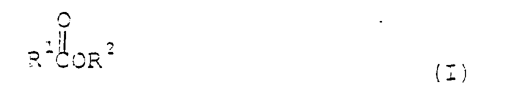 dans laquelle R¹ représente un groupe alkyle ou alcényle à chaîne droite ou ramifié ayant de 5 à 23 atomes de carbone ou un groupe phényle substitué avec un groupe alkyle ayant de 5 à 23 atomes de carbone ; et R² représente un groupe alkyle à chaîne droite ou ramifié ayant de 1 à 4 atomes de carbone,
avec une éthanolamine ou un dérivé d'éthanolamine représenté par la formule (II) :
R³NHCH₂CH₂OH (II)
dans laquelle R³ représente un atome d'hydrogène ou un groupe alkyle à chaîne droite ou ramifiée ayant de 1 à 5 atomes de carbone,
pour former un éthanolamide d'acide gras représenté par la formule (III) : 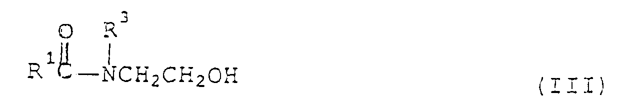 dans laquelle R¹ et R³ sont comme défini ci-dessus ; de faire réagir l'éthanolamide d'acide gras avec de l'oxyde d'éthylène pour obtenir un amidoéther représenté par la formule (IV) : 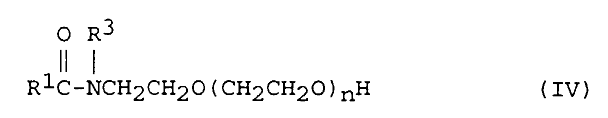 dans laquelle R¹ et R³ sont comme défini ci-dessus ; et n représente un nombre situé dans la plage de 0,5 à 20 en moyenne;
et de faire réagir l'amidoéther avec un acide monohalogénoacétique ou un sel d'un acide monohalogénoacétique représenté par la formule (V) :
XCH₂COOM (V)
dans laquelle X représente un atome d'halogène ; et M représente un atome d'hydrogène, un métal alcalin, un métal alcalino-terreux, un groupe ammonium, une alcanolamine ou un acide aminé basique,
en présence d'un alcali solide, de manière à produire le mélange d'agents tensioactifs contenant un acide amidoéther carboxylique ou un sel de celui-ci, représenté par la formule (VI) : 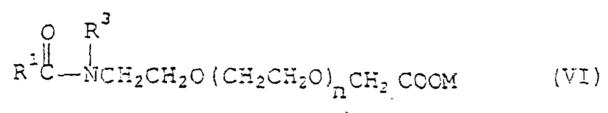 dans laquelle R¹, R³, M et n sont comme défini ci-dessus ;
le rapport de mélange de l'acide amidoéther carboxylique ou d'un sel de celui-ci (VI) sur l'amidoéther (IV), (VI) / (IV) étant dans la plage de 99,9 / 0,1 à 1 / 9 en poids.

2. Procédé selon la revendication 1, dans lequel R¹ est un groupe alkyle ou alcényle à chaîne droite ou ramifié ayant de 7 à 17 atomes de carbone.

3. Procédé selon la revendication 1, dans lequel R³ est un atome d'hydrogène.

4. Procédé selon la revendication 1, dans lequel n est un nombre situé dans la plage de 0,5 à 10 en moyenne.

5. Procédé selon la revendication 1, dans lequel R¹ est un groupe alkyle à chaîne droite ayant de 7 à 17 atomes de carbone ; R² est un groupe méthyle ; R³ est un atome d'hydrogène ; et n est un nombre dans la plage de 0,5 à 5 en moyenne.

6. Procédé selon la revendication 1, dans lequel la réaction de l'ester alkylique d'acide gras représenté par la formule (I) et de l'éthanolamine ou du dérivé de l'éthanolamine représenté par la formule (II) s'effectue à une température de 60 à 120 °C sous une pression de 1,33 - 1033 mbars (1 à 760 mm de Hg).

7. Procédé selon la revendication 1, dans lequel la réaction de l'éthanolamide d'acide gras représenté par la formule (III) et de l'oxyde d'éthylène se fait à une température ne dépassant pas 150 °C.

8. Procédé selon la revendication 1, dans lequel l'acide monohalogénoacétique ou un sel de celui-ci représenté par la formule (V) est utilisé dans un rapport molaire de 0,1 à 3,0 par rapport à l'amidoéther représenté par la formule (IV).

9. Procédé selon la revendication 8, dans lequel la réaction de l'amidoéther représenté par la formule (IV) et de l'acide monohalogénoacétique ou d'un sel de cet acide, représenté par la formule (V) est mise en oeuvre à une température de 50 à 90 °C.

10. Procédé selon la revendication 1, dans lequel l'acide monohalogénoacétique ou un sel de cet acide représenté par la formule (V) et l'alcali solide sont ajoutés par portions divisées, pour réagir avec l'amidoéther représenté par la formule (IV).
